(19) Europäisches Patentamt
European Patent Office
Office européen des brevets

(11) **EP 2 908 121 A1**

(12) **EUROPÄISCHE PATENTANMELDUNG**

(43) Veröffentlichungstag:
   **19.08.2015   Patentblatt 2015/34**

(51) Int Cl.:
   **G01N 27/12** *(2006.01)*       **B82Y 15/00** *(2011.01)*
   **B82Y 30/00** *(2011.01)*

(21) Anmeldenummer: **15000410.9**

(22) Anmeldetag: **11.02.2015**

(84) Benannte Vertragsstaaten:
   **AL AT BE BG CH CY CZ DE DK EE ES FI FR GB
   GR HR HU IE IS IT LI LT LU LV MC MK MT NL NO
   PL PT RO RS SE SI SK SM TR**
   Benannte Erstreckungsstaaten:
   **BA ME**

(30) Priorität: **14.02.2014   DE 102014002077**

(71) Anmelder: **Karlsruher Institut für Technologie
   76131 Karlsruhe (DE)**

(72) Erfinder:
   • **Sommer, Martin
      76227 Karlsruhe (DE)**
   • **Augustin, Martin
      76356 Weingarten (DE)**

(74) Vertreter: **Müller-Boré & Partner
   Patentanwälte PartG mbB
   Friedenheimer Brücke 21
   80639 München (DE)**

(54) **Gassensor und Verfahren zur Detektion von Gasen**

(57)   Gassensor, ausgelegt zur Detektion von Gasen mit einer Mehrzahl von Teilsensoren, die jeweils ein gassensitives Material mit einem bestimmten, messbaren elektrischen Widerstand aufweisen. Dabei weist zumindest einer der Teilsensoren Nanofasern als gassensitives Material auf. Die Nanofasern sind so ausgebildet, dass der elektrische Widerstand der Nanofasern durch Lichtbestrahlung veränderbar ist. Eine Lichtquelle (3) reduziert den elektrischen Widerstand der Nanofasern durch Lichtbestrahlung.

Figur 1

**Beschreibung**

Die Erfindung betrifft einen Gassensor zur Detektion von Gasen sowie ein Verfahren zur Detektion von Gasen.

Ein herkömmlicher Gassensor ist zum Beispiel bekannt unter dem Namen KAMINA, der Karlsruher Mikronase. Das Dokument EP 0 769 141 betrifft einen Gassensor, der zum Detektieren von Gerüchen dient und dafür eine Mehrzahl von Teilsensoren aufweist, die sich alle leicht voneinander unterscheiden. Jeder der Teilsensoren weist eine Schicht aus gassensitivem Material auf, zum Beispiel eine Schicht aus einem oder mehreren halbleitenden Oxiden, wie zum Beispiel Zinndioxid. Der elektrische Widerstand der Schicht aus Zinndioxid ändert sich, wenn die Schicht in Kontakt mit einem reduzierenden oder oxidierenden Gas steht. Diese Änderung des elektrischen Widerstands wird gemessen und ausgewertet.

Jeder der Teilsensoren unterscheidet sich dabei leicht von den anderen Teilsensoren, zum Beispiel durch unterschiedlich dicke Beschichtungen mit einem zusätzlichen Material, wie z.B. $SiO_2$. Da die Empfindlichkeit des Teilsensors gegenüber dem zu detektierenden Gas abhängig ist von der Schichtdicke, reagieren die unterschiedlichen Teilsensoren mit einer unterschiedlich starken Widerstandsänderung auf zu detektierende Gase. Dadurch sind die unterschiedlichen Teilsensoren des Gassensors dazu geeignet, unterschiedliche Gasgemische zu detektieren und zum Beispiel in "Gerüche" einzuordnen, weswegen ein solcher Gassensor auch als "Nase" bezeichnet wird.

Die gassensitive Schicht aus Zinndioxid muss dabei "aktiviert" werden, d.h. die gassensitive Schicht muss sich in einem Zustand befinden, in dem sich der elektrische Widerstand der gassensitiven Schicht in einer starken Abhängigkeit von dem Gasgemisch befindet, das die gassensitive Schicht umgibt. Zur Aktivierung ist es daher notwendig, die gassensitive Schicht zu erhitzen. Dabei werden Aktivierungstemperaturen von über 200°C oder über 250°C benötigt.

Solch herkömmliche Gassensoren weisen mehrere Nachteile auf. Neben dem konstruktiven Mehraufwand, den das Vorsehen einer Heizung bedingt, sind die Betriebskosten des Gassensors hoch, da der Gassensor nur in einem stark erhitzten Zustand einsatzfähig ist. Weiterhin ist der Widerstand der Teilsensoren stark abhängig von der Temperatur, weswegen eine sehr genaue und aufwendige Temperaturregelung notwendig ist, um die Teilsensoren auf eine vorbestimmte Temperatur einzustellen. Insgesamt ist der Energieverbrauch eines herkömmlichen Gassensors so hoch, dass ein Batteriebetrieb des Gassensors nahezu unmöglich ist. Dies erschwert den Einsatz eines solchen Gassensors als z. B. Rauchmelder.

Aus dem Dokument US 8,434,647 ist es bekannt, anstelle der Metalloxidschicht Nanofasern zu verwenden. Zur Aktivierung der Nanofasern als gassensitives Material ist eine Temperatur von zumindest 200°C notwendig.

Sowohl die Gassensoren mit der Metalloxidschicht als auch die Gassensoren mit den Nanofasern sind aufwendig zu konditionieren, entweder durch einen über die Heizung gesteuerten, über die Teilsensoren anliegenden Temperaturgradienten oder durch unterschiedlich starke Nanofaserdichten bzw. -dicken oder unterschiedliche Beschichtungsdicken. Diese Konditionierung der unterschiedlichen Teilsensoren ist sowohl aufwendig als auch ungenau.

Der Erfindung liegt die Aufgabe zugrunde, einen verbesserten Gassensor zum Detektieren von Gasen bereitzustellen, insbesondere einen Gassensor, der zumindest einen der eingangs beschriebenen Nachteile abschwächt.

Diese Aufgabe wird durch die Gegenstände der unabhängigen Ansprüche gelöst. Bevorzugte Ausführungsformen sind Gegenstände der abhängigen Ansprüche.

Ein Aspekt betrifft einen Gassensor, der zur Detektion von Gasen ausgelegt ist und eine Mehrzahl von Teilsensoren aufweist, die jeweils ein gassensitives Material mit einem bestimmten messbaren elektrischen Widerstand aufweisen. Dabei weist zumindest einer der Teilsensoren Nanofasern als gassensitives Material auf. Die Nanofasern sind so ausgebildet, dass der elektrische Widerstand der Nanofasern durch Lichtbestrahlung veränderbar ist. Die Lichtbestrahlung reduziert den elektrischen Widerstand der Nanofasern und ersetzt die bei herkömmlichen Sensoren genutzte Aktivierung der Fasern durch hohe Temperaturen.

Der Gassensor dient insbesondere zur Detektion von reduzierenden oder oxidierenden Gasen, während er ungeeignet zur Detektion von inerten Gasen bzw. Edelgasen ausgebildet sein kann. Die Teilsensoren des Gassensors können sich jeweils leicht voneinander unterscheiden. Dabei unterscheiden sich die Teilsensoren in ihrer Empfindlichkeit voneinander, d.h. die einzelnen Teilsensoren ändern ihren elektrischen Widerstand unterschiedlich stark, wenn sie in Kontakt zu einem bestimmten Gas bzw. Gasgemisch stehen. Damit ist der Gassensor zu einer "Geruchsdetektion" bzw. zur Detektion von unterschiedlichen Gasen und/oder Gasgemischen in unterschiedlichen Zusammensetzungen geeignet.

Zum Auswerten der einzelnen gemessenen elektrischen Widerstände kann ein Verfahren vorgesehen sein, das auf einem mathematischen Algorithmus beruht. Demnach wird eine gemessene Konstellation, z. B. ein gemessenes "Muster" von elektrischen Widerständen an den unterschiedlichen Teilsensoren, mit vorgespeicherten Konstellationen bzw. "Mustern" verglichen. Durch ein Speichern von Geruchsmustern kann der Gassensor trainiert und/oder für bestimmte Gerüche sensibilisiert werden.

Der elektrische Widerstand der gassensitiven Materialien der Teilsensoren ist messbar. Dazu kann der durch den Teilsensor fließende Strom gemessen werden und/oder die am Teilsensor anliegende Spannung. Eine Messung des elektrischen Widerstands umfasst eine Messung der elektrischen Leitfähigkeit des gassensitiven Materials.

Zur Messung des elektrischen Widerstands kann der Gassensor eine Mehrzahl von Elektroden aufweisen, von denen zumindest eine Elektrode in elektrischem Kontakt zu zumindest einem der Teilsensoren steht, so dass der elektrische Widerstand des gassensitiven Materials des zumindest einen Teilsensors messbar ist. Die Elektroden dienen zum elektrischen Kontaktieren der Teilsensoren. Insbesondere können alle Elektroden in einem elektrischen Kontakt zu zumindest einem Teilsensor ausgebildet sein. Vorteilhafterweise befindet sich das gassensitive Material jedes Teilsensors in elektrischem Kontakt zu zwei Elektroden, so dass der elektrische Widerstand des Teilsensors durch den Stromfluss zwischen diesen beiden Elektroden messbar ist. Die einzelnen Elektroden können dabei zum Beispiel in einem Grenzbereich zweier benachbarter Teilsensoren so angeordnet sein, dass zwei benachbarte Teilsensoren von derselben Elektrode kontaktiert werden. Dies reduziert die insgesamt benötigte Anzahl von Elektroden, da eine Elektrode zur Messung des elektrischen Widerstandes zweier unterschiedlicher, benachbarter Teilsensoren verwendbar ist.

Als gassensitives Material zumindest eines der Teilsensoren, bevorzugt aller Teilsensoren, sind Nanofasern vorgesehen. Die Nanofasern können aus einem Metalloxid ausgebildet sein, das mit Gasgemischen in der Umgebung chemisch reagiert. Die Nanofasern können aus einem oder mehreren halbleitenden Metalloxid(en) ausgebildet sein, z.B. aus $SnO_2$ und/oder anderen Metalloxiden, deren Bandlücke zwischen Valenz- und Leitungsband so gering ist, dass eine Anregung mittels Licht bzw. UV-Licht erfolgen kann. Dazu zählen zum Beispiel $WO_3$, $ZnO$, $TiO_2$, $V_2O_5$ oder $In_2O_3$. Die Durchmesser der Nanofasern sind dabei zwischen 10 nm und 2000 nm stark. Bevorzugt sind die Durchmesser der meisten Nanofasern zwischen 50 nm und 1000 nm dick, d.h. die Durchmesser von über 90% der Nanofasern. Dabei weist jeder Teilsensor zumindest so viele Nanofasern auf, dass die zur Messung des elektrischen Widerstandes des Teilsensors vorgesehenen Elektroden über die Nanofasern miteinander in einem elektrischen Kontakt stehen. Alternativ oder zusätzlich zu den Nanofasern können auch Nanotubes verwendet werden.

Anstelle oder zusätzlich zu einer Heizung, wie bei den aus dem Stand der Technik bekannten Sensoren vorgesehen, weist der Gassensor eine Lichtquelle zur gezielten Beleuchtung der Nanofasern auf. Durch Bestrahlung der Nanofasern mit Licht der Lichtquelle wird der elektrische Widerstand der Nanofasern reduziert. Die Lichtquelle kann so ausgerichtet sein, dass die Lichtemission der Lichtquelle auf die Teilsensoren gerichtet ist. Die Aktivierung der gassensitiven Materialien erfolgt bei dem Gassensor somit durch Lichtbestrahlung. Das Licht muss dabei nicht aus dem sichtbaren Spektrum sein, sondern es kann sich um nicht-sichtbares Licht handeln, insbesondere um UV-Licht.

Die Wellenlänge der Lichtbestrahlung kann dabei auf das Material der Nanofasern abgestimmt sein, so dass die Energie der einfallenden Photonen dazu geeignet ist, den elektrischen Widerstand der Nanofasern zu senken. Bei Verwendung von Nanofasern aus einem halbleitenden Material kann z.B. eine Wellenlänge der Lichtbestrahlung gewählt werden, die einem Photonenenergiewert zwischen 50% und 150% der Bandlücke entspricht.

Die Lichtquelle kann die Teilsensoren entweder direkt bestrahlen, oder das Licht kann mittels einer Glasfaser, Spiegeln und/oder Prismen so umgeleitet werden, dass die Teilsensoren bestrahlt werden. Die Lichtquelle bzw. der Glasfaserausgang ist dabei bevorzugt zwischen 1 mm und 10 cm beabstandet von den Teilsensoren angeordnet, besonders bevorzugt zwischen 5 mm und 2 cm beabstandet.

Durch die Verwendung einer Lichtquelle zur Aktivierung des Gassensors entfällt die Notwendigkeit, eine Heizung zur Aktivierung der Teilsensoren vorzusehen. Zwar kann eine Heizung zusätzlich vorgesehen sein, z. B. für eine Feineinstellung der Empfindlichkeit der einzelnen Teilsensoren, jedoch ist eine Heizung durch den Einsatz der Lichtbestrahlung nicht notwendig zum Betrieb des Gassensors. Durch die Aktivierung der Teilsensoren mittels Lichtbestrahlung wird eine Heizung überflüssig. Dies bewirkt eine deutliche Energiereduzierung im Betrieb des Gassensors. Insbesondere kann der Gassensor in einem Batteriebetrieb betrieben werden.

Die Verwendung von Nanofasern anstelle einer Schicht aus Metalloxid als gassensitives Material bewirkt aufgrund der vergrößerten Oberfläche eine bessere analytische Leistung. Somit spart der Gassensor sowohl Energie als auch Kosten ein.

In einer Ausführungsform ist der elektrische Widerstand der Nanofasern durch die Lichtbestrahlung um zumindest 50% reduziert, verglichen mit dem elektrischen Widerstand der Nanofasern ohne Lichtbestrahlung durch die Lichtquelle. Bevorzugt wird der elektrische Widerstand durch die Lichtbestrahlung um zumindest 90% reduziert, so dass Gase leichter detektiert werden können. Je niedriger der elektrische Widerstand der Nanofasern ausgebildet ist, desto sensibler reagiert das gassensitive Material auf reduzierende und oxidierende Gase in unmittelbarer Umgebung der Nanofasern. Eine Reduktion des elektrischen Widerstands der Nanofasern entspricht einem Aktivieren des Gassensors und erhöht die Empfindlichkeit des Gassensors zur Detektion von Gasen.

In einem Ausführungsbeispiel ist die Lichtquelle als UV-Lichtquelle ausgebildet. Die Aktivierung der Nanofasern erfolgt dabei durch Lichtbestrahlung mit UV-Licht. UV-Licht weist eine Wellenlänge von 10 nm bis 400 nm auf. Bevorzugt erfolgt eine Lichtbestrahlung mit Licht mit einer Wellenlänge von 300 nm bis 400 nm. Versuche haben gezeigt, dass durch die Lichtbestrahlung mit UV-Licht der Widerstand der Nanofasern besonders stark reduziert werden kann. Deswegen eignet sich UV-Licht besonders gut zur Aktivierung der Teilsensoren. Die Lichtquelle kann als UV-LED ausgebildet sein, die Licht besonders energieeffizient und genau regelbar abstrahlt.

In einer Ausführungsform sind die Teilsensoren auf maximal 100°C erhitzt, bevorzugt auf maximal 50 °C erhitzt. Besonders bevorzugt wird der Gassensor bei Raumtemperatur derart betrieben, dass die Aktivierung der Nanofasern im Wesentlichen unabhängig von der Umgebungstemperatur ist. Der Gassensor kann insbesondere bei Raumtemperatur ohne eine Heizung aktiviert werden. Das Betreiben des Gassensors bei diesen geringen Temperaturen ist besonders energiesparend und deswegen kostengünstig, insbesondere im Dauerbetrieb.

In einer Ausführungsform erfolgt die Lichtbestrahlung durch die Lichtquelle mit einer Leistung in einem Bereich von 0,1 mW/cm$^2$ bis 250 mW/cm$^2$. Die Lichtbestrahlung kann bevorzugt mit einer Leistung in einem Bereich von 2,0 mW/cm$^2$ bis 10 mW/cm$^2$ erfolgen, besonders bevorzugt in einem Bereich von 2,5 mW/cm$^2$ bis 10 mW/cm$^2$. $^2$. Die Lichtbestrahlung kann auch mit einer Leistung in einem Bereich von 0,1 mW/cm$^2$ bis 50 mW/cm$^2$ erfolgen, besonders bevorzugt in einem Bereich von 0,1 mW/cm$^2$ bis 10 mW/cm$^2$, besonders bevorzugt in einem Bereich von 0,4 mW/cm$^2$ bis 4 mW/cm$^2$. Schon die Lichtbestrahlung mit dieser geringen Leistung ist ausreichend, um den elektrischen Widerstand zur Aktivierung des Gassensors ausreichend abzusenken. Damit wird es ermöglicht, einen besonders energiesparenden Gassensor bereitzustellen, der günstig im Betrieb ist. Damit kann der Gassensor sowohl Energie einsparen, da er bei niedrigen Temperaturen betrieben werden kann, als auch weil die Lichtbestrahlung besonders wenig Leistung benötigt. Insbesondere kann der Gassensor bei diesem geringen Energieverbrauch dazu ausgebildet und vorgesehen sein, mittels einer Batterie als Energiequelle betrieben zu werden.

In einer Ausführungsform weist der Gassensor eine im Wesentlichen zusammenhängende Schicht von Nanofasern als gassensitives Material auf, wobei die Schicht aus Nanofasern durch eine Mehrzahl von Elektroden so in Felder unterteilt ist, dass ein Feld der Schicht aus Nanofasern einem Teilsensor entspricht. Im Wesentlichen zusammenhängende Schicht bedeutet dabei, dass die Schicht so aus Nanofasern ausgebildet ist, dass sich eine Art Gewebe aus Nanofasern bildet. Die Nanofasern können dabei Knotenpunkte, Kreuzungspunkte und/oder Berührungspunkte miteinander aufweisen. Somit kann selbst aus Nanofasern einer maximalen Länge von einigen 100 μm eine im Wesentlichen zusammenhängende Schicht aus Nanofasern ausgebildet werden, die sich über mehrere Quadratmillimeter bzw. Quadratzentimeter erstreckt. Die Elektroden zur Messung des elektrischen Widerstands der Teilsensoren können dabei parallel zu der Schicht aus Nanofasern ausgebildet sein, so dass sie die Schicht aus Nanofasern in Felder unterteilen. Dabei bilden die Elektroden Feldergrenzen der einzelnen Felder, die den Teilsensoren entsprechen.

In dieser Ausführungsform können sich die Teilsensoren durch die jeweilige Dichte der Schicht aus Nanofasern voneinander unterscheiden, so dass die Teilsensoren in Kontakt zu einem zu detektierenden Gas unterschiedliche elektrische Widerstände aufweisen.

Alternativ oder zusätzlich zu dieser Ausführungsform können die einzelnen Teilsensoren auch unterschiedlich stark und mit unterschiedlicher Wellenlänge beleuchtet werden, so dass sie sich ausreichend voneinander unterscheiden, um auf Gase unterschiedlich stark zu reagieren. Die unterschiedliche Ausbildung der einzelnen Teilsensoren voneinander, also die unterschiedlich starke Empfindlichkeit der einzelnen Teilsensoren für Gase, ermöglicht ein "Riechen" von Gasgemischen.

In einer Ausführungsform ist der Gassensor als batteriebetriebener Gassensor ausgebildet. Dabei kann sowohl die Leistung zum Betreiben der Lichtquelle als auch die Leistung zum Betreiben der Teilsensoren durch die Batterie als Energiequelle bereitgestellt werden. Der Gassensor kann somit als ausschließlich batteriebetriebener Gassensor ausgebildet sein.

In einer Ausführungsform ist der Gassensor als Bestandteil eines Brandsensors ausgebildet. Der Brandsensor dient zur Früherkennung und zur Warnung bei Bränden. Im Unterschied zu Brandsensoren, die einen Rauchgehalt in der Umgebungsluft überprüfen, kann der Gerüche detektierende Brandsensor Brände früher erkennen und klassifizieren. Vor der detektierbaren Entwicklung von Rauch bilden sich oftmals detektierbare Gerüche aus.

Ein Aspekt betrifft ein Verfahren zum Detektieren von Gasen mit den Schritten:

- Bereitstellen einer Mehrzahl von Teilsensoren, die jeweils ein gassensitives Material aufweisen,
- Messen des elektrischen Widerstands des gassensitiven Materials der Teilsensoren,
- Verwenden von Nanofasern als gassensitives Material der Mehrzahl von Teilsensoren und
- Reduzieren des elektrischen Widerstands der Nanofasern durch Lichtbestrahlung.

Das Verfahren kann den weiteren Schritt aufweisen:

- Mathematisches Auswerten der gemessenen elektrischen Widerstände der Nanofasern zur Klassifizierung eines Gasgemisches.

Eine solche mathematisches Auswerten kann zum Beispiel mittels einer "Linearen Diskriminanz Analyse", kurz: LDA, erfolgen. Die Auswertung kann mittels eines programmierbaren Prozessors erfolgen. Durch LDA lässt sich ein gemessenes Muster von elektrischen Widerständen als "Geruch" mit einem vorher gemessenen und gespeicherten, also einem "antrainierten" Muster vergleichen und diesem zuordnen. Dadurch kann ein Gasgemisch klassifiziert werden. Dazu kann ein Gassensor, der die Mehrzahl von Teilsensoren aufweist, zunächst in einem Lernprozess angelernt werden, wobei er mehreren Gerüchen ausgesetzt wird. Der Gassensor kann in der Lernphase mit Gasgemischen unterschiedlicher Zusammensetzung konfrontiert werden, wobei die elektrischen Widerstände der einzelnen Teilsensoren gemessen und so gespeichert werden, dass bestimmte Muster von elektrischen Widerständen bestimmten Gasgemischen zugeordnet sind. Wird der Gassensor im Einsatz mit einem Gasgemisch konfrontiert, das zumindest einem antrainierten und vorgespeicherten Gasgemisch ähnelt, erfolgt mittels mathematischer Auswertung eine Zuordnung des aktuellen gemessenen Gasgemisches (also des aktuell gemessenen Musters der elektrischen Widerstände der einzelnen Teilsensoren) zu dem ähnlichsten vorgespeicherten Gasgemisch. Diese Auswertungsmethode erlaubt eine anwendungspezifische Konditionierung des Gassensors auf bestimmte antrainierte Gerüche, z.B. zur Verwendung als Brandsensor.

Ein Aspekt betrifft das Verwenden eines Gassensors gemäß des ersten Aspekts zum Durchführen des Verfahrens gemäß des zweiten Aspekts.

Ein Aspekt betrifft ein Verfahren zum Herstellen eines Gassensors gemäß dem ersten Aspekt. Dabei werden in einem ersten Schritt Nanofasern auf einem Substrat aus schwarzem Silizium hergestellt, z.B. mittels HTC (High Temperature Condensation). Die Nanofasern werden von dem schwarzen Silizium abgelöst und in einem zweiten Schritt als gassensitives Material auf den Gassensor aufgebracht, z.B. in einer Lösung, die sich anschließend verflüchtigt und die Nanofasern auf dem Gassensor zurücklässt.

Schwarzes Silizium ist ein kristallines Silizium mit einer Oberflächenmodifikation. Durch hochenergetischen Beschuss mit Ionen und/oder ultrakurzen Laserpulsen werden nadelförmige Strukturen auf der Oberfläche des Siliziums ausgebildet, die die Reflexion des Substrats stark vermindern. Die nadelförmigen Strukturen können als Keime beim Wachsen der Nanofasern dienen. Das schwarze Silizium kann mit Standardätzprozessen hergestellt sein

Die Erfindung wird im Folgenden anhand von in Figuren gezeigten Ausführungsbeispielen näher beschrieben. Einzelne Merkmale der in den Figuren gezeigten Ausführungsformen lassen sich mit anderen erfindungsgemäßen Ausführungsformen kombinieren. Es zeigen:

Fig. 1    eine Fotographie eines Gassensors,
Fig. 2    eine Fotographie eines Chips mit Nanofasern in einer Keramikkarte,
Fig. 3    eine REM-Aufnahme von Nanofasern auf einem Substrat aus schwarzem Silizium,
Fig. 4    eine REM-Aufnahme einer Schicht aus Nanofasern, die Elektroden miteinander verbindet,
Fig. 5    in einem Diagramm die Abhängigkeit einer Sensorempfindlichkeit von der Lichtbestrahlung,
Fig. 6A    in einem Diagramm die Abhängigkeit des elektrischen Widerstandes einer hochdichten Schicht aus Nanofasern von der Wellenlänge der Lichtbestrahlung bei verschiedenen Betriebstemperaturen,
Fig. 6B    in einem Diagramm die Abhängigkeit des elektrischen Widerstandes einer niedrigdichten Schicht aus Nanofasern von der Wellenlänge der Lichtbestrahlung bei verschiedenen Betriebstemperaturen,
Fig. 7A    in einem Diagramm die Abhängigkeit der Empfindlichkeit einer hochdichten Schicht aus Nanofasern von der Wellenlänge der Lichtbestrahlung und der Betriebstemperatur,
Fig. 7B    in einem Diagramm die Abhängigkeit der Empfindlichkeit einer niedrigdichten Schicht aus Nanofasern von der Wellenlänge der Lichtbestrahlung und der Betriebstemperatur,
Fig. 8A    in einem Diagramm die Reaktionszeit, die ein Gassensor mit einer hochdichten Schicht aus Nanofasern benötigt, um bei Lichtbestrahlung einen um zumindest 90% reduzierten elektrischen Widerstand aufzuweisen, in Abhängigkeit von der Wellenlänge der Lichtbestrahlung und der Betriebstemperatur,
Fig. 8B    in einem Diagramm die Reaktionszeit, die ein Gassensor mit einer niedrigdichten Schicht aus Nanofasern benötigt, um bei Lichtbestrahlung einen um zumindest 90% reduzierten elektrischen Widerstand aufzuweisen, in Abhängigkeit von der Wellenlänge der Lichtbestrahlung und der Betriebstemperatur,
Fig. 9A    in einem Diagramm die Erholzeit eines Gassensors mit einer hochdichten Schicht aus Nanofasern, in Abhängigkeit von der Wellenlänge der Lichtbestrahlung und der Betriebstemperatur und
Fig. 9B    in einem Diagramm die Erholzeit eines Gassensors mit einer niedrigdichten Schicht aus Nanofasern, in Abhängigkeit von der Wellenlänge der Lichtbestrahlung und der Betriebstemperatur.

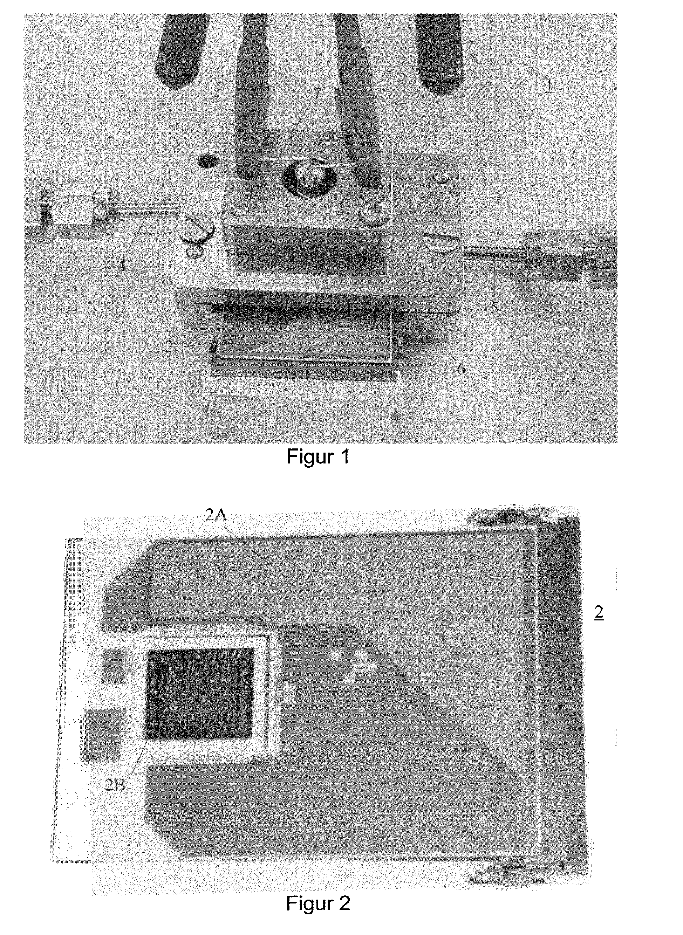

Fig. 1 zeigt die Fotographie eines Gassensors 1 zum Detektieren von Gasen. Der Gassensor weist einen Gaseinlass 4 und einen Gasauslass 5 auf. Der Gaseinlass 4 leitet Gas in ein Gehäuse 6 des Gassensors ein, aus dem der Gasauslass 5 das zu detektierende Gas auslässt.

In das Gehäuse 6 ist ein Chip 2 eingeführt, der vergrößert in der in Fig. 2 gezeigten Fotographie gezeigt ist. Der Chip 2 weist eine Keramikkarte 2A und einen Nanofaserbereich 2B auf. Der Chip ist im KAMINA Design ausgeführt (Karlsruher Mikronase). In diesem Design kann der Chip 2 in verschiedene Gassensoren eingeführt werden, die dem KAMINA Standard entsprechen, z.B. in den in Figur 1 gezeigten Gassensor 1.

Der Gassensor 1 weist weiterhin eine Lichtquelle 3 auf, die als eine LED ausgebildet ist, die Licht im UV-Bereich abstrahlt. Über elektrische Anschlüsse 7 wird die LED mit Strom versorgt. Über den Stromfluss durch die Lichtquelle 3 kann eine Lichtbestrahlung von Teilsensoren des Gassensors 1 reguliert werden.

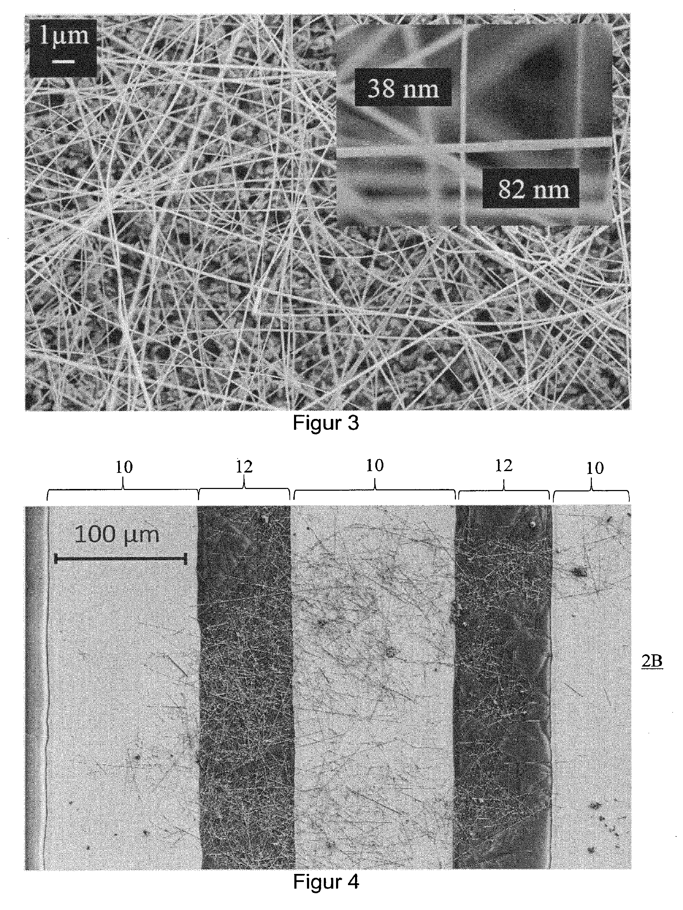

Der Nanofaserbereich 2B ist in einer stark vergrößerten Aufnahme in **Fig. 4** gezeigt. Die Nanofasern des Nanofaserbereichs 2B sind aus zumindest einem halbleitenden Metalloxid aufgebaut, zum Beispiel aus $SnO_2$. Unter Lichtbestrahlung durch die Lichtquelle 3 sinkt der elektrische Widerstand der Nanofasern.

Bei der Herstellung des Chips 2 wird bevorzugt darauf geachtet, den Herstellungsprozess aus Kostengründen möglichst einfach zu gestalten und gleichzeitig eine hohe Leistungsfähigkeit des Gassensors 1 zu ermöglichen.

Herstellung der Nanofasern

Die Nanofasern können mittels eines eigenen Herstellungsverfahrens hergestellt sein und anschließend auf den Chip übertragen werden. Zur kostengünstigen Herstellung der Nanofasern ist eine Methode bevorzugt, die auf einem HTC Verfahren beruht, was als Kurzform kurz für High Temperature Condensation steht. HTC ist ein schnelles und vergleichsweise einfaches Verfahren, qualitativ hochwertige Nanofasern zu erzeugen **Fig. 3** zeigt in einer REM-Aufnahme Nanofasern, die auf einem Substrat aus schwarzem Silizium ausgebildet sind, das durch standardisierte Ätzverfahren hergestellt sein kann. Im Vergleich zu anderen Ablagerungsverfahren, wie zum Beispiel der MBE, kurz für Molecular Beam Epitaxy, sind bei der Herstellung der Nanofasern mittels HTC keine teuren Ultra-Hoch-Vakuum (kurz:UHV)-Instrumente notwendig.

Die Ausbildung der Nanofasern mittels HTC verursacht einen niedrigen Ordnungsgrad der Nanofasern, was für den Gassensor von Vorteil ist. Im HTC Verfahren kann z.B. SnO Pulver als Precursor in einen Aluminiumtiegel in einem Quarzrohr eingebracht werden, das in einem horizontalen Heizrohr angeordnet ist. Der Druck und der Fluss der Prozessgase Ar und $O_2$ kann durch ein Gasmischsystem reguliert werden. Alle Prozessparameter können mittels eines PC gesteuert und protokolliert werden.

Das Substrat aus schwarzem Silizium weist vorzugsweise eine Oberfläche von einigen wenigen Quadratzentimetern auf, zum Beispiel eine Oberfläche von ca. 1 cm x 4 cm.

Der HTC Herstellungsprozess kann bei einer Prozesstemperatur zwischen 880°C und 1000°C durchgeführt werden. Das schwarze Silizium kann mittels eines RIE Prozesses (kurz für Reactive Ion Etching) eines Siliziumwafers mit $SF_6$ und $O_2$ bereitgestellt werden. Dadurch wird eine optisch schwarze Oberfläche bereitgestellt, die von "Nadeln" bedeckt ist. Die Nadeln weisen einen durchschnittlichen Nadelspitzendurchmesser von ungefähr 200 nm auf. Der durchschnittliche Abstand zwischen den Nadelspitzen beträgt ungefähr 5 $\mu$m.

Beim diesem HTC Herstellungsverfahren kann das Quarzrohr evakuiert werden und ein Gasfluss des Argongases von 50 sccm/min eingestellt werden. Während des Aufheizens kann 15 Minuten lang die Temperatur bei 300°C gehalten werden, um zunächst Keime für die Nanofasern auszubilden. Anschließend kann ein Druck von ca. 400 mbar eingestellt werden. Bei Erreichen der Prozesstemperatur von 1000°C kann für zwei Stunden ein Sauerstofffluss von 0,5 sccm/min eingestellt werden.

Nach diesem Verfahren wird die aus Nadeln bestehende Oberfläche des schwarzen Siliziums zu einer fast vollständig geschlossenen Oberfläche umgewandelt. Nach einem Abkühlprozess bildet sich eine weiße uniforme Schicht aus Nanofasern auf dem Substrat, die bis zu einigen Millimetern lang sind.

Während des Herstellungsprozesses formen die Nadeln des schwarzen Siliziumsubstrats eine Siliziumdioxidschicht, in der $SnO_2$ Kristalle mit Durchmessern zwischen 10 nm und einigen Mikrometern teilweise eingebettet sind, welche als Keime der Nanofasern wirken. Die Nanofasern wachsen als loses Netzwerk mit lediglich einigen wenigen Knotenpunkten bzw. Verbindungen. Der Durchmesser einzelner Nanofasern beträgt in der Regel durchschnittlich 60 nm an der Substratoberfläche und bis zu 1000 nm in den oberen Lagen. Mit einer Länge von mehreren 100 $\mu$m bis zu einigen Millimetern werden zumeist ultralange Nanofasern ausgebildet. Da die Nadelspitzen des schwarzen Siliziums besonders gute Keimzellen zum Wachsen der Nanofasern bieten, ist schwarzes Silizium besonders gut geeignet als Substratmaterial des Gassensors.

Die in Fig. 3 gezeigten Nanofasern können anschließend mit einem Skalpell vom Substrat gelöst werden und in einem Ultraschallbad behandelt werden.

Gassensor

Eine solche Nanofaserlösung, die so gelöste und behandelte Nanofasern enthält, kann auf ein Substrat eines Chips aufgebracht werden. Figur 2 zeigt den Nanofaserbereich 2B des Chips 2, auf den eine solche Nanofaserlösung aufgebracht wurde. Die Lösung verdampft und die Nanofasern bleiben als im Wesentlichen zusammenhängende Schicht auf dem Substrat des Nanofaserbereichs 2B zurück.

Der Nanofaserbereich 2B des Chips 2 kann gemäß KAMINA Standard 9 mm x 8 mm groß ausgebildet sein. In einem Ausführungsbeispiel weist der Nanofaserbereich 39 Platinelektroden auf, jeweils mit einer Breite von ca. 120 $\mu$m und einer Länge von 4,5 mm, die ca. 70 $\mu$m voneinander beabstandet sind. Zwischen den 39 Platinelektroden sind so 38 Felder bzw. Sensorsegmente als Teilsensoren des Gassensors 1 ausgebildet. Jede Einzelne der Platinelektroden (mit Ausnahme der beiden Randelektroden) dient zum Messen des elektrischen Widerstands zweier benachbarter Teilsensoren. Durch diese Aufteilung ist die aktive Sensoroberfläche zusammen mit den Elektroden ca. 33,75 mm² groß ausgebildet.

Die Elektroden können auf dem Siliziumsubstrat mittels eines hochfrequenten Magnetronsputtering und einer Schattenmaskentechnik ausgebildet werden. Zwei Temperatursensoren an den Längsenden des als Sensorarray ausgebildeten Gassensors sowie vier mäanderförmige Heizelektroden können auf der den Nanofasern abgewandten Seite des Siliziumsubstrats ausgebildet sein. Die Heizelektroden können einzeln ansteuerbar ausgebildet sein, so dass ein Temperaturgradient auf der Fläche des Chips 2 ausgebildet werden kann. Zur elektrischen Kontaktierung ist der Nanofaserbereich 2B mit einer standardisierten Keramikkarte 2A gebondet.

Die unterschiedlichen Teilsensoren können eine gemeinsame Schicht aus Nanofasern aufweisen. Die Nanofasern aus Metalloxid dienen dabei als gassensitives Material des Gassensors 1. Die Dichte dieser Schicht aus Nanofasern kann dabei für die Teilsensoren unterschiedlich ausgebildet sein, so dass die Dichte der Schicht aus Nanofasern von einem Teilsensor zu einem benachbarten Teilsensor ab- oder zunimmt.

Um einen solchen Dichtegradienten auf der Chipoberfläche auszubilden, kann ein Tropfen von ca. 10 µl Nanofaserlösung auf einer ersten Hälfte des Nanofaserbereichs 2B aufgebracht werden, z.B. den für die Elektroden 1 bis 19 vorbestimmten Bereich des Gassensors. Der Tropfen kann anschließend auf die zweite Hälfte des Nanofaserbereichs 2B fließen, z.B. den für die Elektroden 20 bis 39 vorgesehenen Bereich des Chips. Durch die Fließbewegung des Tropfens der Lösung aus Nanofasern stellt sich automatisch ein Dichtegradient auf der Chipoberfläche ein. Allgemein kann ein Dichtegradient durch eine Fließbewegung einer Nanofaserlösung auf einem Substrat ausgebildet werden.

Dabei ist die Dichte der Nanofasern auf allen Teilsensoren groß genug, zwei aufeinanderfolgende Elektroden elektrisch miteinander zu kontaktieren. Nachdem die Flüssigkeit aus der Nanofaserlösung verdampft ist, bleibt auf dem Substrat eine im Wesentlichen zusammenhängende Schicht aus Nanofasern zurück. Eine entsprechende Schicht ist in Fig. 4 gezeigt.

Fig. 4 zeigt eine hochauflösende REM-Aufnahme eines Teilbereichs des Gassensors 1. In dem gezeigten Nanofaserbereich 2B bedeckt die Schicht aus Nanofasern zwischen 20 und 30 Prozent der dargestellten Oberfläche zwischen den einzelnen Elektroden. Damit weist der in Fig. 4 gezeigte Bereich der Schicht aus Nanofasern eine relativ hohe Dichte auf. Die Dichte der Nanofasern in der Schicht kann von Teilsensor zu Teilsensor soweit abnehmen, dass in einem anderen Bereich lediglich eine einzelne Nanofaser den elektrischen Kontakt zwischen den Elektroden des Teilsensors bereitstellt. Insbesondere kann ein Dichtegradient der Schicht aus Nanofasern so ausgebildet sein, dass zwischen 1% und 30% der Oberfläche der Teilsensoren mit Nanofasern bedeckt ist. Dabei kann an einem ersten Ende der Schicht zwischen 1% und 10% der Oberfläche mit Nanofasern bedeckt sein, während am gegenüberliegenden zweiten Ende zwischen 20% und 30% der Oberfläche mit Nanofasern bedeckt ist. Dazwischen nimmt die Dichte der Nanofasern graduell zu.

In dem in **Fig. 4** gezeigten Teilbereich des Nanofaserbereichs 2B des Gassensors 1 sind streifenförmige Elektroden 10 parallel zueinander auf einem Substrat so ausgebildet, dass sie von einer im Wesentlichen zusammenhängenden Schicht aus Nanofasern bedeckt sind. Zwischen den streifenförmigen Elektroden 10 sind Sensorbereiche 12 ausgebildet, die elektrodenfrei sind, d.h. auf denen keine Elektroden angeordnet sind. In diesen Sensorbereichen 12 sind lediglich Nanofasern aus einem Metalloxid wie z.B. $SnO_2$ angeordnet, die die Elektroden 10 miteinander elektrisch verbinden. Eine einzelne Elektrode 10 dient dabei zum Messen des elektrischen Widerstandes an den zwei Sensorbereichen 12, die an die jeweilige Elektrode 10 angrenzen. Somit dient jede Elektrode, bis auf die erste und letzte Elektrode, zur Messung des elektrischen Widerstandes zweier Sensorbereiche 12. Jeder der Sensorbereiche 12 ist einem Teilsensor des Chips 2 zugeordnet und die im Sensorbereich 12 angeordneten Nanofasern wirken als gassensitives Material des zugeordneten Teilsensors. Wie in Fig. 4 gezeigt, unterteilen die Elektroden 10 das gassensitive Material, nämlich die im Wesentlichen zusammenhängende Schicht aus Nanofasern, in einzelne Felder, von denen eines der Felder als Sensorbereich 12 einem Teilsensor entspricht.

Die einzelnen Teilsensoren können sich in ihrer elektrischen Leitfähigkeit voneinander unterscheiden, indem sie entweder eine unterschiedliche Dichte von Nanofasern aufweisen, unterschiedlich stark beleuchtet sind (z.B. mit unterschiedlichen Strahlungsleistungen), mit Licht einer unterschiedlichen Wellenlänge beleuchtet werden (z.B. aufgeteilt mittels eines Prismas), und/oder unterschiedlich stark erhitzt werden (z.B. mittels eines Temperaturgradienten zwischen Heizelektroden). Somit kann der Chip 2 mit mehreren Teilsensoren versehen sein, die sich alle leicht voneinander unterscheiden. Diese Unterschiedlichkeit der Sensitivitäten der einzelnen Teilsensoren dient als Basis für das Detektieren von Gasen, wobei die Oberflächen der Nanofasern mit dem zu detektierenden Gas reagieren. Das zu detektierende Gas kann dabei zum Beispiel ein oxidierendes oder reduzierendes Gas sein. Die Reaktion des gassensitiven Materials mit dem zu detektierenden Gas ändert die elektrische Leitfähigkeit bzw. den elektrischen Widerstand der Teilsensoren jeweils unterschiedlich stark. Gewisse Gerüche können diesem Sensor somit als ein "Muster" elektrischer Widerstände der unterschiedlichen Teilsensoren antrainiert werden, so dass detektierte Gerüche mittels einer mathematischen Analyse antrainierten Mustern zugeordnet werden können. Für die mathematische Analyse eignet sich z.B. eine LDA-Analyse, was kurz für Lineare Diskriminanz Analyse steht. Die Muster werden in Abhängigkeit von der Unterschiedlichkeit der einzelnen Teilsensoren trainiert. So können zum Beispiel die Teilsensoren mit unterschiedlichen Lichtmustern so bestrahlt werden, dass sie sich voneinander hinreichend bezüglich ihrer Empfindlichkeit (z.B. Response S, siehe unten) unterscheiden, um eine Geruchsdetektion zu ermöglichen.

Der Chip 2 weist bevorzugt zwischen 8 und 100 Teilsensoren auf, besonders bevorzugt zwischen 20 und 50 Teilsensoren, besonders bevorzugt zwischen 35 und 45 Teilsensoren. Durch eine solche Anzahl von Teilsensoren kann eine hinreichend genaue Detektion von unterschiedlichen Gasen bzw. Gerüchen gewährleistet werden.

Als Lichtquelle 3 des Gassensors 1 ist eine UV-Lichtquelle vorgesehen, zum Beispiel eine LED. In einem Versuch wurde als Lichtquelle 3 eine einstellbare Lichtquelle verwendet, eine Lumatec Superlight 400 mit einer maximalen Strahlungsleistung von ungefähr 2,4 W/cm$^2$. Weiterhin wurde im Versuch die Empfindlichkeit des Gassensors bei Raumtemperatur, bei 50°C und bei 100°C gemessen.

Der elektrische Widerstand des gassensitiven Materials der Teilsensoren ist abhängig von der Intensität der Lichtbestrahlung, der Wellenlänge der Lichtbestrahlung, der Temperatur des Gassensors sowie der Ausgestaltung des einzelnen Sensors, zum Beispiel der Dichte der Nanofasern. Ein besonders starker Effekt zeigt sich bei einer Bestrahlung im UV-Bereich mit Licht einer Wellenlänge von 320 nm bis 400 nm. Die Aktivierung der Teilsensoren kann mittels einer Empfindlichkeit als Response S angegeben werden. Dabei gilt:

$$S = 1 - R_{on} / R_{off}.$$

$R_{on}$ bezeichnet dabei den elektrischen Widerstand des Teilsensors unter Lichtbestrahlung, während $R_{off}$ den elektrischen Widerstand des Teilsensors ohne Lichtbestrahlung angibt. Bei Lichtbestrahlung mit UV-Licht mit einer Strahlungsdichte von ca. 2,7 mW/cm$^2$ wird bereits eine Response S von 92% erreicht.

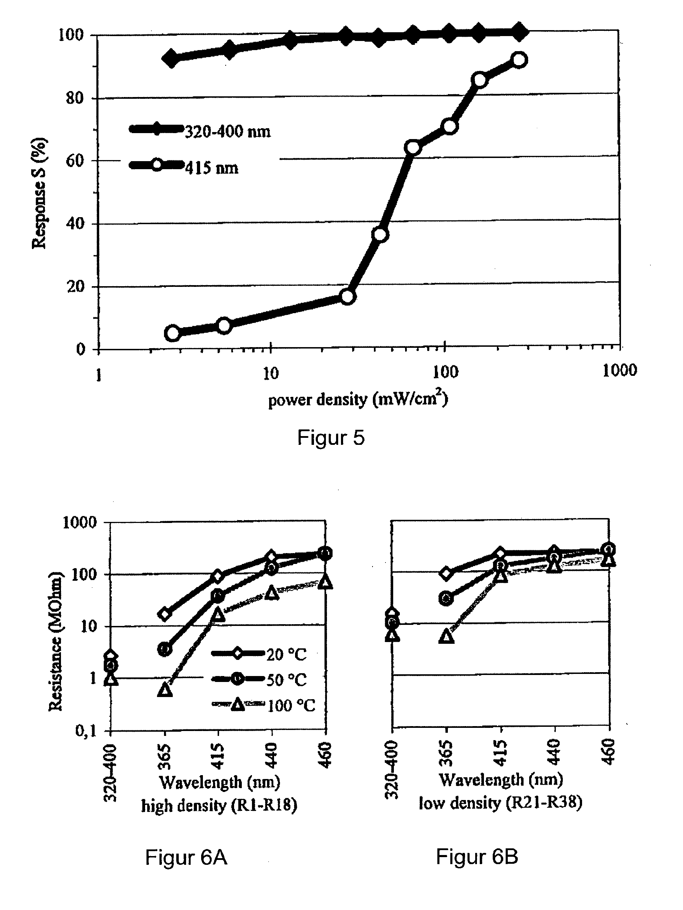

Die Response S eines Versuchs ist in **Fig. 5** in einem Diagramm gezeigt, das die Response S in Prozent in Abhängigkeit von der Strahlungsleistung und der Wellenlänge der Lichtbestrahlung angibt. Dabei ist die Strahlungsleistung der Lichtbestrahlung an der X-Achse logarithmisch aufgetragen. Unter einer Lichtbestrahlung mit Licht der Wellenlänge zwischen 320 nm und 400 nm wird bereits bei einer sehr geringen Strahlungsleistung (ab ca. 0,5 mW/cm$^2$) eine gute Response S von über 90% an den Teilsensoren bewirkt, was einer Aktivierung der Teilsensoren entspricht. Bei Lichtbestrahlung mit Licht einer Wellenlänge von 415 nm wird eine wesentlich höhere Strahlungsleistung benötigt, um die Teilsensoren zu aktivieren. Bei Lichtbestrahlung mit einer Wellenlänge von 415 nm sind Strahlungsleistungen von über 100 mW/cm$^2$ notwendig, um eine Response S von 90% oder mehr zu bewirken. Deswegen erfolgt die Lichtbestrahlung des Chips 2 bevorzugt mit Licht einer Wellenlänge von 320 nm bis 400 nm.

Bei Bestrahlung mit UV-Licht mit einer Strahlungsleistung von über 80 mW/cm$^2$ wird eine Sättigung der Aktivierung erzielt, während bei Bestrahlung mit sichtbarem Licht mit eine Strahlungsleistung von 195 mW/cm$^2$ ± 18% lediglich eine Response von 80% bewirkt wird.

**Figuren 6A und 6B** zeigen in Diagrammen die gemessene Abhängigkeit des elektrischen Widerstandes der Nanofasern in MOhm von der Wellenlänge der Lichtbestrahlung, sowie der Temperatur und der Dichte der Nanofasern.

**Fig. 6A** zeigt die Abhängigkeit des elektrischen Widerstandes von der Wellenlänge der Lichtbestrahlung und der Betriebstemperatur bei einem Gassensor mit einer im Wesentlichen zusammenhängenden Schicht aus Nanofasern mit einer relativ hohen Dichte (Oberflächenbedeckung zwischen 20 bis 30%).

**Fig. 6B** zeigt in einem Diagramm die Abhängigkeit des Widerstandes der Schicht aus Nanofasern von der Wellenlänge des Bestrahlungslichts und der Betriebstemperatur für eine Schicht aus Nanofasern mit einer niedrigen Dichte, nämlich mit weniger als 10% Oberflächenbedeckung.

In den Diagrammen zeigt sich, dass der Widerstand deutlich von der Bestrahlungswellenlänge abhängt. So ist der Widerstand der Nanofasern bei Bestrahlung mit Licht einer Wellenlänge von 365 nm deutlich geringer als bei Bestrahlung mit Licht einer Wellenlänge von 460 nm. Allgemein ist der Widerstand bei Lichtbestrahlung mit Licht einer Wellenlänge von 415 nm zwischen vier und zehn mal höher als der Widerstand bei Bestrahlung mit einer Lichtwellenlänge von 365 nm. Deswegen weist der Gassensor bevorzugt eine Lichtquelle für UV-Licht mit einer Wellenlänge unter 400 nm auf.

Ein Erhitzen des Gassensors resultiert in einer weiteren Minimierung des elektrischen Widerstandes unterhalb eines MOhms bei Erhitzung auf über 100°C und Bestrahlung mit Licht einer Wellenlänge von 365 nm (vgl. Fig. 6A).

Allgemein weisen die Schichten aus Nanofasern mit einer höheren Dichte einen geringeren elektrischen Widerstand auf als diejenigen mit einer geringen Dichte aus Nanofasern (vgl. Fig. 6A mit Fig. 6B). Die elektrischen Widerstände bei Raumtemperatur waren im Versuch oberhalb der Messgrenze von 340 MOhm, bei 50°C zwischen 240 MOhm und 340 MOhm und bei 100°C stabil bei ca. 180 MOhm. Diese elektrischen Widerstände beziehen sich auf die Widerstände ohne Lichtbestrahlung. Unter Lichtbestrahlung werden die elektrischen Widerstände erheblich reduziert. Die Empfindlichkeit des Sensors, die Response S, nimmt mit abnehmender Temperatur und zunehmender Lichtenergie zu.

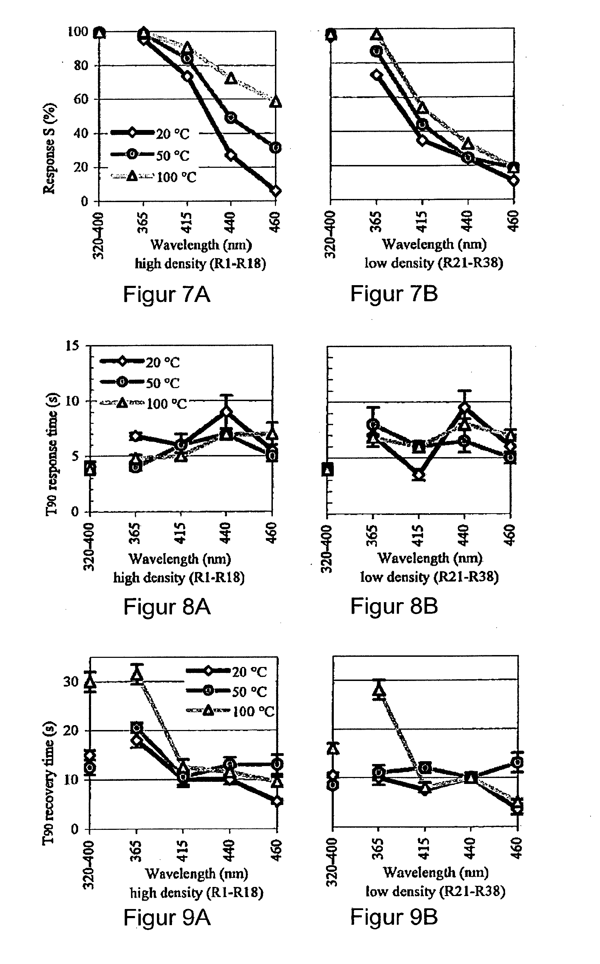

So zeigen **Figuren 7A und 7B** in jeweils einem Diagramm die Empfindlichkeit "Response S" aufgetragen in Prozent in Abhängigkeit von der Wellenlänge der Lichtbestrahlung sowie der Temperatur der Nanofasern. Dabei zeigt das Diagramm in Fig. 7A die Abhängigkeit der Response S für eine Schicht aus Nanofasern mit hoher Dichte im Vergleich zu dem in Fig. 7B gezeigten Diagramm für eine Schicht aus Nanofasern von geringerer Dichte.

Allgemein ist die Response S größer für eine Schicht mit höherer Dichte (Fig. 7A). Die in Fig. 7B gezeigte Abhängigkeit der Empfindlichkeit des Gassensors von der Betriebstemperatur ist für Gassensoren mit einer geringen Dichte der Schicht aus Nanofasern nahezu vernachlässigbar. Solche Gassensoren bzw. Teilsensoren des Gassensors mit einer solch geringen Dichte funktionieren bei Raumtemperatur nahezu genauso gut wie im erhitzten Zustand bei z.B. 100°C. Schichten aus Nanofasern mit einer relativ hohen Dichte können aus Hunderten von Nanofasern bestehen, die die Elektroden an den Teilsensoren über mehrere hundert Mikrometer miteinander verbinden. Schichten aus Nanofasern einer relativ geringen Dichte bestehen aus weniger Nanofasern, bei denen der Kontakt zwischen den Elektroden zur Messung des elektrischen Widerstandes des gassensitiven Materials durch einige vereinzelte Nanofasern hergestellt werden kann.

Bei Lichtbestrahlung mit sichtbarem Licht bei Raumtemperatur sinkt die Response S, also die Empfindlichkeit des Sensors, auf unter 20% bei 460 nm Wellenlänge des Bestrahlungslichts. Die Empfindlichkeit Response S bei UV-Licht kann größer als 99% für Teilsensoren mit Schichten aus Nanofasern einer hohen Dichte sein, während selbst bei Raumtemperatur auch bei geringen Schichtdichten unter UV-Lichtbestrahlung eine Response S von über 95% erzielbar ist.

**Figuren 8A und 8B** zeigen in jeweils einem Diagramm die Abhängigkeit der T90 Antwortzeit von der Wellenlänge der Lichtbestrahlung und der Temperatur der Nanofasern, einmal für hohe Dichten (siehe Fig. 8A) und einmal für niedrige Dichten (siehe Fig. 8B). Die T90 Antwortzeit ist definiert als die Zeit, die der Gassensor benötigt, um einen Widerstandsabfall vom Ausgangswiderstand (ohne Lichtbestrahlung) zum Minimalwiderstand (mit Lichtbestrahlung) von 90% durchzuführen. Somit ist die T90 Antwortzeit ein Maß für die Aktivierungszeit des Sensors. Diese Aktivierungszeit ist in den Figs. 8A und 8B für unterschiedliche Wellenlängen der Lichtbestrahlung gezeigt.

**Figuren 9A und 9B** zeigen in jeweils einem Diagramm eine T90 Erholzeit für unterschiedliche Wellenlängen. Nachdem die Lichtbestrahlung der Teilsensoren abgeschaltet wird, kann am Gassensor eine T90 Erholzeit gemessen werden. Die T90 Erholzeit ist definiert als diejenige Zeit, die das gassensitive Material nach Abschalten der Lichtbestrahlung benötigt, um ein erstes Sättigungsplateau nach einem anfänglichen starken Anstieg der elektrischen Widerstandsfähigkeit zu erreichen. Die T90 Aktivierungszeiten (vgl. Figuren 8A und 8B) liegen üblicherweise zwischen 4 und 10 Sekunden. Die T90 Erholzeiten liegen zwischen 8 und 30 Sekunden. Der Abfall des elektrischen Widerstands beginnt unmittelbar nach dem Einschalten der Lichtquelle, so dass unabhängig von der T90 Antwortzeit bereits nach 2 Sekunden die Detektion von Gasen möglich ist.

**Bezugszeichenliste**

| | |
|---|---|
| 1 | Gassensor |
| 2 | Chip mit Nanofasern |
| 2A | Keramikkarte |
| 2B | Nanofaserbereich |
| 3 | Lichtquelle |
| 4 | Gaseinlass |
| 5 | Gasauslass |
| 6 | Gehäuse |
| 7 | elektrische Anschlüsse |
| 10 | Elektrode |
| 12 | Sensorbereich |

**Patentansprüche**

1. Gassensor, ausgelegt zur Detektion von Gasen mit

   - einer Mehrzahl von Teilsensoren, die jeweils ein gassensitives Material mit einem bestimmten, messbaren elektrischen Widerstand aufweisen, wobei
   - zumindest einer der Teilsensoren Nanofasern als gassensitives Material aufweist,
   - die Nanofasern so ausgebildet sind, dass der elektrische Widerstand der Nanofasern durch Lichtbestrahlung veränderbar ist und
   - eine Lichtquelle (3) den elektrischen Widerstand der Nanofasern durch Lichtbestrahlung reduziert.

2. Gassensor nach Anspruch 1, wobei die Lichtquelle (3) als UV-Lichtquelle ausgebildet ist.

3. Gassensor nach Anspruch 1 oder 2, wobei die Lichtbestrahlung durch die Lichtquelle mit einer Leistung in einem Bereich von 0,1 mW/cm$^2$ bis 10 mW/cm$^2$ erfolgt.

4. Gassensor nach einem der vorangegangenen Ansprüche, wobei der elektrische Widerstand der Nanofasern durch die Lichtbestrahlung um zumindest 50% reduziert ist.

5. Gassensor nach einem der vorangegangenen Ansprüche, wobei die Teilsensoren auf maximal 100°C erhitzt sind.

6. Gassensor nach einem der vorangegangenen Ansprüche, wobei der Gassensor als batteriebetriebener Gassensor ausgebildet ist.

7. Gassensor nach einem der vorangegangenen Ansprüche, mit einer im Wesentlichen zusammenhängenden Schicht aus Nanofasern als gassensitives Material, die durch eine Mehrzahl von elektrischen Elektroden (10) so in Felder (12) unterteilt ist, dass ein Feld (12) der Schicht aus Nanofasern einem Teilsensor entspricht.

8. Gassensor nach Anspruch 7, wobei sich die Teilsensoren durch eine Dichte der Nanofasern auf den jeweiligen Feldern (12) voneinander so unterscheiden, dass die Teilsensoren in Kontakt zu einem zu detektierenden Gas unterschiedliche elektrische Widerstände aufweisen.

9. Brandsensor mit einem Gassensor nach einem der vorangegangenen Ansprüche.

10. Verfahren zum Detektieren von Gasen mit den Schritten

   - Bereitstellen einer Mehrzahl von Teilsensoren, die jeweils ein gassensitives Material aufweisen,
   - Verwenden von Nanofasern als gassensitives Material der Mehrzahl von Teilsensoren,
   - Reduzieren des elektrischen Widerstands der Nanofasern durch Lichtbestrahlung und
   - Messen des elektrischen Widerstands des gassensitiven Materials der Teilsensoren.

11. Verfahren nach Anspruch 10, wobei der elektrische Widerstand der Nanofasern durch Lichtbestrahlung mit einer UV-Lichtquelle mit einer Leistung in einem Bereich von 0,1 mW/cm$^2$ bis 10 mW/cm$^2$ erfolgt.

12. Verfahren nach Anspruch 10 oder 11, mit dem Schritt:

   - mathematisches Auswerten der gemessenen elektrischen Widerstände der Nanofasern zum Bestimmen der Zusammensetzung eines Gasgemisches.

13. Verwenden eines Gassensors nach einem der Ansprüche 1 bis 8 zum Durchführen des Verfahrens nach einem der Ansprüche 10 bis 12.

14. Verfahren zum Herstellen eines Gassensors nach einem der Ansprüche 1 bis 8, wobei Nanofasern auf einem Substrat aus schwarzem Silizium mittels High Temperature Condensation hergestellt werden, die Nanofasern von dem schwarzen Silizium abgelöst werden und die Nanofasern als gassensitives Material auf den Gassensor (1) aufgebracht werden.

15. Verfahren nach Anspruch 14, wobei die Nanofasern in einer Lösung auf den Gassensor (1) aufgebracht werden, die sich verflüchtigt und die Nanofasern auf dem Gassensor (1) zurücklässt.

Figur 1

Figur 2

Figur 3

Figur 4

Figur 5

Figur 6A

Figur 6B

Figur 7A

Figur 7B

Figur 8A

Figur 8B

Figur 9A

Figur 9B

Europäisches Patentamt
European Patent Office
Office européen des brevets

**EUROPÄISCHER RECHERCHENBERICHT**

Nummer der Anmeldung

EP 15 00 0410

| | **EINSCHLÄGIGE DOKUMENTE** | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (IPC) |
| X | E. ZAMPETTI ET AL: "UV Assisted Chemical Sensor Based on Electrospun Titania nanofibers Working at Room Temperature", PROCEDIA ENGINEERING, Bd. 47, 9. September 2012 (2012-09-09), Seiten 912-915, XP055183120, ISSN: 1877-7058, DOI: 10.1016/j.proeng.2012.09.295 | 1-7,9-15 | INV. G01N27/12 B82Y15/00 B82Y30/00 |
| Y | * Zusammenfassung; Abbildungen 1-4 * <br> * Seite 913, Absatz 3 - Absatz 4 * <br> * Seite 914, Absatz 1 * <br> ----- | 8 | |
| X | JIAN GONG ET AL: "UV-Light-Activated ZnO Fibers for Organic Gas Sensing at Room Temperature", JOURNAL OF PHYSICAL CHEMISTRY C, Bd. 114, Nr. 2, 23. Dezember 2009 (2009-12-23), Seiten 1293-1298, XP055183085, ISSN: 1932-7447, DOI: 10.1021/jp906043k | 1-7, 9-11,13 | |
| Y <br> A | * Zusammenfassung; Abbildungen 1-9 * <br> * Seite 1293, rechte Spalte, Absatz 1 * <br> * Seite 1295, linke Spalte, Absatz 1 * <br> ----- | 8 <br> 12,14,15 | RECHERCHIERTE SACHGEBIETE (IPC) <br><br> G01N <br> B82Y |
| X <br><br> Y <br> A | WO 2013/095730 A2 (UNIV UTAH RES FOUND [US]) 27. Juni 2013 (2013-06-27) <br> * Anspruch 31; Abbildungen 1-13 * <br> * Seite 2, Zeile 2 - Zeile 10 * <br> * Seite 10, Zeile 9 - Zeile 15 * <br> * Seite 23, Zeile 18 - Zeile 19 * <br> ----- <br> -/-- | 1,10,12, 13 <br> 8 <br> 2-7,9, 11,14,15 | |

Der vorliegende Recherchenbericht wurde für alle Patentansprüche erstellt

| Recherchenort | Abschlußdatum der Recherche | Prüfer |
|---|---|---|
| München | 16. April 2015 | Gangl, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
.......................................................................
& : Mitglied der gleichen Patentfamilie, übereinstimmendes Dokument

EPO FORM 1503 03.82 (P04C03)

Europäisches
Patentamt

European
Patent Office

Office européen
des brevets

**EUROPÄISCHER RECHERCHENBERICHT**

Nummer der Anmeldung

EP 15 00 0410

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | BONEX WAKUFWA MWAKIKUNGA ET AL: "Towards an electronic nose based on nano-structured transition metal oxides activated by a tuneable UV light source", 2011 IEEE SENSORS PROCEEDINGS : LIMERICK, IRELAND, 28 - 31 OCTOBER 2011, IEEE, PISCATAWAY, NJ, 28. Oktober 2011 (2011-10-28), Seiten 1109-1112, XP032093591, DOI: 10.1109/ICSENS.2011.6127403 ISBN: 978-1-4244-9290-9 * Zusammenfassung; Abbildungen 1-3 * * Seite 3, linke Spalte, Absatz 4 - Seite 4, linke Spalte, Absatz 2 * ----- | 1-15 | |
| A | R. WIERZBICKI ET AL: "Black silicon maskless templates for carbon nanotube forests", MICROELECTRONIC ENGINEERING, Bd. 104, 8. Dezember 2012 (2012-12-08), Seiten 110-113, XP055183398, ISSN: 0167-9317, DOI: 10.1016/j.mee.2012.11.019 * Zusammenfassung; Abbildungen 1,2,5 * * Seite 111, linke Spalte, Absatz 2 - Seite 113, linke Spalte, Absatz 2 * ----- -/-- | 1-15 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 16. April 2015 | Gangl, Martin |

EPO FORM 1503 03.82 (P04C03)

Europäisches Patentamt
European Patent Office
Office européen des brevets

**EUROPÄISCHER RECHERCHENBERICHT**

Nummer der Anmeldung

EP 15 00 0410

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| A | J.D. PRADES ET AL: "Equivalence between thermal and room temperature UV light-modulated responses of gas sensors based on individual SnO2 nanowires", SENSORS AND ACTUATORS B: CHEMICAL, Bd. 140, Nr. 2, 21. Mai 2009 (2009-05-21), Seiten 337-341, XP055183402, ISSN: 0925-4005, DOI: 10.1016/j.snb.2009.04.070 * Zusammenfassung; Abbildungen 1,2 * * Seite 338, linke Spalte, Absatz 2 - Seite 339, linke Spalte, Absatz 2 * ----- | 1-15 | |
| X,P | AUGUSTIN MARTIN ET AL: "UV assisted room temperature gas recognition with SnO2 nanowires", 14TH IEEE INTERNATIONAL CONFERENCE ON NANOTECHNOLOGY, IEEE, 18. August 2014 (2014-08-18), Seiten 574-577, XP032690961, DOI: 10.1109/NANO.2014.6968060 [gefunden am 2014-11-26] | 1-13 | |
| Y,P | * das ganze Dokument * ----- | 14,15 | |
| Y,P | MARTIN AUGUSTIN ET AL: "UV-VIS sensor system based on SnO2 nanowires", SENSORS AND ACTUATORS A: PHYSICAL, Bd. 210, 25. Februar 2014 (2014-02-25), Seiten 205-208, XP055183391, ISSN: 0924-4247, DOI: 10.1016/j.sna.2014.02.016 * Zusammenfassung; Abbildungen 1-8 * * Seite 205, rechte Spalte, Absatz 2 - Seite 206, rechte Spalte, Absatz 2 * ----- | 14,15 | |

RECHERCHIERTE SACHGEBIETE (IPC)

Der vorliegende Recherchenbericht wurde für alle Patentansprüche erstellt

| Recherchenort | Abschlußdatum der Recherche | Prüfer |
|---|---|---|
| München | 16. April 2015 | Gangl, Martin |

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 15 00 0410

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

16-04-2015

| Im Recherchenbericht angeführtes Patentdokument | Datum der Veröffentlichung | Mitglied(er) der Patentfamilie | Datum der Veröffentlichung |
|---|---|---|---|
| WO 2013095730 A2 | 27-06-2013 | CN 103975237 A<br>EP 2758772 A2<br>US 2014235493 A1<br>WO 2013095730 A2 | 06-08-2014<br>30-07-2014<br>21-08-2014<br>27-06-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0769141 A
- US 8434647 B